# EUROPÄISCHE PATENTSCHRIFT

(11) **EP 3 092 986 B1**
(45) Veröffentlichungstag und Bekanntmachung des Hinweises auf die Patenterteilung: **14.06.2023**
(21) Anmeldenummer: 16168672.0
(22) Anmeldetag: 09.05.2016
(51) Int. Cl.: A61F 9/013, A61F 9/009

(54) **OPHTHALMOLOGISCHE UNTERDRUCKVORRICHTUNG UND PATIENTENINTERFACE**
OPHTHALMOLOGICAL VACUUM DEVICE AND PATIENT INTERFACE
DISPOSITIF OPHTALMOLOGIQUE A VIDE ET INTERFACE PATIENT

(30) Priorität: 11.05.2015 EP 15167073
(43) Veröffentlichungstag der Anmeldung: 16.11.2016
(73) Patentinhaber: Ziemer Ophthalmic Systems AG, 2562 Port (CH)
(72) Erfinder: Rathjen, Christian, 28197 Bremen (DE); Studer, Thomas, 2000 Neuchatel (CH)
(74) Vertreter: Rentsch Partner AG

(56) Entgegenhaltungen:
- WO-A1-2008/150330
- WO-A1-2012/041350
- US-A1- 2002 120 285
- US-A1- 2002 198 553

## Beschreibung

### TECHNISCHES GEBIET

Die vorliegende Erfindung betrifft Vorrichtungen und Verfahren im Bereich der Ophthalmologie. Die Erfindung betrifft insbesondere Unterdruckvorrichtungen zur Kopplung eines Patienteninterfaces an ein Patientenauge sowie Patienteninterfaces. Die Erfindung betrifft ferner Verfahren zur Kopplung eines Patienteninterfaces an ein Patientenauge.

### STAND DER TECHNIK

Zur Behandlung und/oder Diagnose von Augengewebe ist der Einsatz von Strahlungsgeneratoren, insbesondere Lasern, bekannt. Entsprechende Vorrichtungen wie ophthalmologischen Lasereinrichtungen weisen beispielsweise ein Basisgerät mit einer Laser-Lichtquelle zur Erzeugung von Laserpulsen, beispielsweise Femtosekundenlaserpulse, sowie einen Applikationskopf mit einem Projektionsobjektiv auf, der zur Behandlung mit dem Patientenauge gekoppelt wird. Der Applikationskopf kann, beispielsweise über einen Gelenkarm, beweglich mit dem Basisgerät verbunden sein, wobei der Gelenkarm zugleich der optischen Strahlführung von der Laser-Lichtquelle zum Applikationskopf dienen kann. Eine entsprechende Anordnung ist beispielsweise in der EP 1731120 offenbart. Des Weiteren gibt es Geräte, bei denen der Applikationskopf im Basisgerät integriert ist oder bei denen andere Vorrichtungsanordnungen vorgesehen sind.

Die mechanische und optische Kopplung des Applikationskopfes an das Patientenauge, beispielsweise an die Hornhaut und/oder Sklera des Patientenauges, erfolgt über ein Patienteninterface, wobei das Patienteninterface einen transparenten Kontaktkörper umfassen kann, durch welchen die aus dem Projektionsobjektiv austretenden Laserpulse geleitet werden und der durch mechanischen Kontakt mit der Hornhaut diese bezüglich des Patienteninterface und des Projektionsobjektives fixiert. Alternativ zur Kopplung mittels eines Kontaktkörpers kann eine Flüssigkeitskopplung vorgesehen werden, wobei sich zwischen Hornhaut und Projektionsobjektiv eine Koppelflüssigkeit, beispielsweise physiologische Kochsalzlösung, befindet. Entsprechende Patienteninterfaces sind beispielsweise aus der WO 2012031277 bekannt. Die Kopplung des Patienteninterface an das Patientenauge kann mittels Vakuum und einer Unterdruckkavität des Patienteninterfaces erfolgen. Die Unterdruckkavität ist typischerweise ein auf die Hornhaut aufgesetzten Saugring. Die meisten Saugringe haben zwei Dichtlippen. Die Lippen können auf der Sklera, der Sklera und der Hornhaut oder nur auf der Hornhaut angebracht sein. Weiterhin gibt es Varianten, die nur einen Ring besitzen und über dem ganzen Auge ein Vakuum erzeugen, oder Varianten, die aus mehreren Saugkammern/Saugnäpfen bestehen. Der Saugring ist die gebräuchlichste Methode der Befestigung, es gibt jedoch auch andere bekannte Lösungen. In jedem Fall erfolgt die Kopplung an das Patientenauge durch ein Vakuum bzw. ein Unterdruck in einer Unterdruckkavität des Patienteninterfaces, wobei die Unterdrucckavität entlang ihres Umfangs dichtend auf dem Patientenauge aufliegt und das Patienteninterface so fluidisch dichtend an das Patientenauge koppelt und gegen die Umwelt abdichtet. Die Erzeugung des Unterdrucks kann durch einen Unterdruckgenerator, insbesondere eine Vakuumpumpe bzw. Unterdruckpumpe, erfolgen. Die Kopplung des Patienteninterfaces an den Applikationskopf erfolgt bei bekannten Systemen beispielsweise mittels Schraubverbindung, Bajonettverschlüssen oder Vakuumkupplungen.

Die US 2002/0120285 A1 offenbart eine Klingenführung für ein ophthalmologisches chirurgisches Instrument, welche mittels Vakuum auf dem Patientenauge fixiert ist und den Kontaktdruck zwischen der Sklera und der Klingenführung misst.

Die US 2002/0198553 A1 offenbart ein Patenteninterface und eine Unterdruckvorrichtung mit fluidische Druckmessung, wobei die Verbindung zum Patienteninterface über eine gemeinsame fluidische Leitung erfolgt.

Die WO 2008/150330 offenbart ein Patienteninterface, welches zur Kopplung mittels Vakuum an das Patientenauge vorgesehen und zweiteilig aufgebaut ist, wobei an einer Koppelstelle zwischen den Teilen Kontaktdrucksensoren angeordnet sind, welche einen Kontaktruck zwischen den Teilen erfassen.

### DARSTELLUNG DER ERFINDUNG

Während der Anwendung ist insbesondere sicherzustellen, dass das Patienteninterface sicher und definiert mit dem Patientenauge verbunden und auf diesem fixiert ist. Eine unsichere oder sich lösende Fixierung hätte zur Folge, dass die vom Applikationskopf ausgehenden diagnostischen oder therapeutischen Strahlen das Patientenauge nicht mehr oder - im schlimmsten Fall - undefiniert bzw. in falscher Weise treffen.

Eine derartige Situation kann beispielsweise auftreten, wenn die die Unterdruckkavität des Patienteninterfaces mit dem Unterdruckgenerator verbindende Leitung blockiert wird. Dies kann z. B. dadurch entstehen, dass der aus der Unterdruckkavität heraus gerichtete Luftstrom beim Aufbau des Unterdrucks Flüssigkeit, z.B. bei Kataraktoperationen verwendete Viscoelastika, oder Sterilabdeckfolie oder Bindegewebe ansaugt, und so die zur Absaugung dienende Unterdruckverbindungsleitung selbst und/oder ihren Anschuss and die Unterdruckkavität, z. B. den Innenraum des Saugrings, ganz oder teilweise blockiert. In diesem Fall kann der Unterdruckgenerator in der Unterdruckkavität des Patienteninterfaces keinen zur sicheren Fixierung ausreichenden Unterdruck aufbauen bzw. aufrechterhalten. Entsprechend zeigt in einer solchen Situation eine typischerweise am Ende der Unterdruckverbindungsleitung angeordnete Druckmesseinrichtung, typischerweise ein Drucksensor, einen Unterdruck an, auch wenn in der Unterdruckkavität kein ausreichender Unterdruck vorhanden ist und das Patienteninterface nicht sicher auf dem Patientenauge fixiert ist oder sich von diesem sogar gelöst hat. Eine vergleichbare Situation entsteht, wenn die Unterdruckverbindungsleitung zwischen Unterdruckgenerator und Patienteninterface im Betrieb abgeknickt wird. Ein derartiger Zustand, bei dem ein ausreichender Unterdruck bzw. ein Vakuum in der Unterdruckkavität nur scheinbar, tatsächlich aber nicht vorhanden ist, wird auch als Pseudovakuum bezeichnet.

Eine weitere typische Fehlerquelle betrifft die Entstehung temporärer oder dauerhafter Leckagen zwischen Unterdruckkavität und Patientenauge oder innerhalb des fluidischen Systems, beispielsweise der Unterdruckverbindungsleitung selbst oder ihrer Anschlussstücke.

Es ist eine Aufgabe der vorliegenden Erfindung, Vorrichtungen und Verfahren bereitzustellen, welche den Stand der Technik hinsichtlich einer sicheren Kopplung zwischen Patienteninterface ist und Patientenauge fortbilden und insbesondere die Situation hinsichtlich der Detektion möglicher Fehlerzustände verbessern. Diese Aufgabe wird in allgemeiner Form durch den Gegenstand der unabhängigen Patentansprüche gelöst. Beispielhafte oder vorteilhafte Ausführungsformen werden durch die abhängigen Patentansprüche sowie die Gesamtoffenbarung des vorliegenden Dokuments definiert.

Gemäss eines ersten Aspekts wird die Aufgabe durch Bereitstellung einer Unterdruckvorrichtung zur Fixierung eines Patienteninterfaces auf einem Patientenauge gelöst. Die Unterdruckvorrichtung umfasst einen Unterdruckgenerator und eine vorrichtungsseitigen Unterdruckschnittstelle zur fluidischen Kopplung des Unterdruckgenerators an eine Unterdruckkavität des Patienteninterfaces. Die Unterdruckkavität wird typischerweise durch den Innenraum eines Saugrings gebildet.

Die Fixierung des Patienteninterfaces auf dem Patientenauge bedeutet im engeren Sinne die Fixierung eines Patienteninterfacekörpers auf dem Patientenauge, beispielsweise auf der Hornhaut und/oder Sklera. Eine dem Patientenauge gegenüberliegenden Seite des Patienteninterfacekörpers ist zur Kopplung eines ophthalmologischen Applikationskopfes ausgelegt. Das Patienteninterface als Ganzes kann weitere Komponenten wie insbesondere Verbindungsleitungen umfassen.

Die Unterdruckvorrichtung umfasst ferner einen fluidischen Drucksensor und eine vorrichtungsseitige Drucksensorschnittstelle zur von der Unterdruckschnittstelle funktionell separaten Kopplung des Drucksensors an das Patienteninterface. Da Druck und Kraft über die Fläche in bekanntem Zusammenhang stehen, kann der Drucksensor technisch auch als Kraftsensor ausgeführt sein. Der Begriff "Drucksensor" ist somit auch als "druckempfindlicher Sensor" bzw. "druckreaktiver Sensor" zu lesen.

Der Drucksensor kann als typischerweise elektrischer Drucksensor grundsätzlich bekannter Art kann mit weiteren Bestandteilen der Unterdruckvorrichtung kombiniert und z. B. in einem gemeinsamen Gehäuse angeordnet sein. In diesem Fall ist die vorrichtungsseitige Drucksensorschnittstelle eine fluidische Schnittstelle. Ferner kann der Drucksensor räumlich separat von weiteren Bestandteilen der Unterdruckvorrichtung, insbesondere separat vom Unterdruckgenerator und/oder der Steuereinrichtung sein. Dann kann der Drucksensor z. B. unmittelbar an dem auf das Patientenauge aufgesetzten Patienteninterfacekörper angeordnet oder ein Bestandteil eines Patienteninterfacekörpers sein. In letzteren Fällen kann die vorrichtungsseitige Sensorschnittstelle z. B. eine elektrische oder faseroptische Schnittstelle sein. Grundsätzlich kann die Unterdruckvorrichtung aus einem einzelnen kompakten Gerät oder aus einer Reihe separater Geräte oder Vorrichtungen mit entsprechender operativer Kopplung bestehen.

Für den Betrieb erfolgt die Kopplung des Drucksensors an das Patienteninterface z. B. derart, dass der Drucksensor mit dem Unterdruck im Innenraum der Unterdruckkavität beaufschlagt wird und diesem misst. In einer alternativen Ausführungsform entsprechend der vorliegenden Offenbarung kann der Drucksensor statt eines fluidischen Drucksensors ein Kontaktdrucksensor und so angeordnet sein, das er mit dem Kontaktdruck bzw. der Kontaktkraft zwischen dem Patienteninterface, insbesondere dem Patienteninterfacekörper, und dem Patientenauge beaufschlagt wird und diesen bzw. diese misst. In jedem Fall misst der Drucksensor im Betrieb einen Druck bzw. eine Kraft, die mit dem Interdruck im Innenraum der Unterdruckkavität in funktionellem Zusammenhang steht.

Die Unterdruckvorrichtung umfasst ferner eine mit dem Unterdruckgenerator und dem Drucksensor oder der vorrichtungsseitigen Drucksensorschnittstelle operativ gekoppelte Steuereinheit, wobei die Steuereinheit dazu ausgelegt ist, den Unterdruckgenerator zur Erzeugung eines Unterdrucks in der Unterdruckkavität anzusteuern. Die Steuereinheit ist ferner dazu ausgelegt, einen durch den Drucksensor ermittelten Druck auszuwerten.

Das Auswerten des Drucks kann insbesondere ein Ermitteln einer fehlerhaften fluidischen Kopplung des Unterdruckvolumens umfassen oder aus einer solchen bestehen. Bei der fehlerhaften fluidischen Kopplung kann es sich um eine fehlerhafte fluidische Kopplung an das Patientenauge und/oder eine fehlerhaft fluidische Kopplung an die Unterdruckvorrichtung bzw. ihren Unterdruckgenerator handeln.

Eine fehlerhafte fluidische Kopplung der Unterdruckkavität an den Unterdruckgenerator und/oder das Patientenauge kann sich insbesondere aus einer Leckage/Undichtigkeit und/oder aufgrund eines Pseudovakuums gemäß vorheriger Darstellung während des Absaugens der Luft aus dem unter Druckvolumen bzw. während des laufenden Betriebs ergeben. Die fehlerhaft fluidische Kopplung hat zur Folge, dass der erforderliche Unterdruck in der Unterdruckkavität zumindest teilweise verloren geht bzw. gar nicht erst korrekt aufgebaut werden kann.

"Fluidische Kopplung" bedeutet im Zusammenhang des vorliegenden Dokuments bevorzugt eine geometrisch definierte fluidische Kopplung, beispielsweise mittels geometrisch definierter Strömungskanäle wie, Schläuche, Rohre und/oder Anschlussstutzen, und nicht eine geometrisch undefinierte fluidische Kopplung über die umgebende Atmosphäre. "Funktionell separate Kopplung" bedeutet, dass die Kopplung unabhängig vom übrigen fluidischen System ist und keine oder lediglich vernachlässigbare fluidischen Wechselwirkungen bestehen. Wie weiter unten näher dargestellt, ergibt sich dies insbesondere mittels eines Anschlusses über separate Strömungskanäle, wie etwa eine separate fluidische Verbindungsleitung und/oder eine direkte Integration des Drucksensors in den Patienteninterfacekörper. Die einzige fluidische Kopplung zwischen Drucksensorschnittstelle und Unterdruckschnittstelle und damit zwischen dem Drucksensor und dem Unterdruckgenerator erfolgt damit über die Unterdruckkavität des Patienteninterfaces.

In einem fluidischen Zusammenhang bedingt eine funktionell separate Kopplung somit eine fluidisch separate Kopplung. Eine funktionell separate Kopplung des Drucksensors an die Unterdruckkavität bedeutet insbesondere, dass zwischen dem Drucksensor und der vorrichtungsseitigen Drucksensorschnittstelle einerseits sowie übrigen fluidische Komponenten der Unterdruckvorrichtung andererseits, insbesondere der vorrichtungsseitigen Unterdruckschnittstelle und dem Unterdruckgenerator, keine fluidische Verbindung besteht. Eine mechanische Integration, z. B. durch einen mit der Unterdruckschnittstelle gemeinsamen Doppelverbinder mit separaten fluidischen Kanälen, ist jedoch nicht ausgeschlossen.

Die funktionell separate oder funktionell unabhängige Kopplung erstreckt sich dabei auf den gesamten fluidischen Pfad zwischen dem Drucksensor und der Unterdruckkavität. Der den Drucksensor mit der Unterdruckkavität verbindende Strömungskanal führt somit fluidisch unabhängig und bevorzugt verzweigungsfrei vom Drucksensor bis zur Unterdruckkavität und mündet unmittelbar in die Unterdruckkavität.

Durch eine funktionell separate Kopplung des Drucksensors an die Unterdruckkavität wird erreicht, dass der Drucksensor - ohne das etwaige Vorliegen weiterer Fehlerfälle wie weiter untern diskutiert - mit dem tatsächlichen Innendruck der Unterdruckkavität oder einer mit diesem korrelierten Druck beaufschlagt wird, auch wenn die fluidische Kopplung über die Unterdruckschnittstelle vollständig oder teilweise blockiert ist.

Der Unterdruckgenerator umfasst typischerweise eine Vakuumpumpe grundsätzlich bekannter Beauart, kann aber zusätzlich oder alternativ auch andere Unterdruck- oder Saugvorrichtungen umfassen, insbesondere ein Unterdruckreservoir in Form eines unterdruck-beaufschlagten Behälters. Optional umfasst die Unterdruckvorrichtung weitere fluidische Komponenten umfassen wie ein oder mehrere Ventile, welche ganz oder teilweise mit der Steuereinheit operativ gekoppelt sein und durch diese gesteuert werden können, sowie Drosseln und Überdruckventile.

Die Steuereinheit ist als elektronische Schaltung in grundsätzlich bekannter Weise realisiert und umfasst typischerweise einen oder mehrere Mikroprozessoren und/oder Mikrokontroller, Speicherbauelemente, sonstige analoge und/oder digital Halbleiterkomponenten, usw. Verfahren und Algorithmen zur Steuerung des Unterdruckgenerators sowie zur Auswertung und Verarbeitung des Drucksensorsignals sind typischerweise ganz oder teilweise in Form von Programmcode für den mindestens einen Mikroprozessor und/oder Mikrokontroller realisiert, können aber auch ganz oder teilweise durch entsprechende Schaltungskomponenten realisiert sein.

Das Patienteninterface kann zur Kopplung an das Patientenauge mittels Koppelflüssigkeit und/oder zur applanierenden Kopplung mittels transparentem Kontaktkörper ausgelegt sein. Im Fall der Flüssigkeitskopplung liegt der für eine sichere Fixierung des Patienteninterfaces erforderliche und durch die Unterdruckvorrichtung herzustellende und aufrechtzuerhaltende Sollunterdruck *p*ₙₒₘ in einem typischen Bereich von beispielsweise -200mbar ...-850mbar, wobei zur Kopplung eines applanierenden Patienteninterfaces typischerweise ein höherer Unterdruck gewählt wird als für ein Patienteninterface mit Flüssigkeitskopplung. Unterdrücke bezeichnen in diesem Dokument Unterdrücke bezogen auf den atmosphärischen Umgebungsdruck. Im stationären Betrieb nach dem Aufbau des Unterdrucks in der Unterdruckkavität sind die Steuereinheit und der Unterdruckgenerator typischer weise zur Ausregelung von Druckschwankungen und zur Aufrechterhaltung des Sollunterdrucks ausgelegt. Die Kopplung des Patienteninterfaces mit einer Therapie- oder Diagnose-Einrichtung, beispielsweise einem Laser-Applikationskopf, kann vor oder nach der Kopplung mit dem Patientenauge erfolgen.

Die Unterdruckschnittstelle und die Drucksensorschnittstelle sind typischerweise als funktionell lösbare und zerstörungsfreie fluidische Koppler, beispielsweise fluidische Steckverbinder, ausgelegt. Die Kopplung des Patienteninterfaces mit einer Therapie- oder Diagnose-Einrichtung, beispielsweise einem Laser-Applikationskopf, kann vor oder nach der Kopplung mit dem Patientenauge erfolgen. Im Fall eines extern von der Unterdruckvorrichtung angeordneten Drucksensors kann die vorrichtungsseitige Drucksensorschnittstelle aber auch eine Datenschnittstelle, beispielsweise eine elektrische oder optische Schnittstelle, sein oder umfassen. Grundsätzlich ist aber auch eine feste und nicht zerstörungsfrei lösbare Kopplung mit einem Patienteninterface möglich.

In typischen Ausführungsformen ist die Steuereinheit mit einer Alarmierungsvorrichtung funktionell gekoppelt oder zur funktionellen Kopplung mit einer Alarmierungsvorrichtung ausgelegt. Die Steuereinheit ist dann ferner dazu ausgelegt, die Alarmierungsvorrichtung im Falle einer detektierten fehlerhaften fluidischen Kopplung der Unterdruckkavität zu aktivieren. Die Alarmierungsvorrichtung kann Bestandteil der Unterdruckvorrichtung und/oder extern von diesem sein. Die Alarmierungsvorrichtung kann typischerweise akustische Signalgeber wie Lautsprecher, Summer oder Sirenen zuzüglich gegebenenfalls erforderlicher Ansteuerschaltungen wie Warnlampen, Leuchtdioden usw. zuzüglich gegebenenfalls vorhandener Ansteuerschaltungen umfassen.

Ferner kann die Steuereinheit zur operativen leitungsgebundenen und/oder drahtlosen Kopplung mit eines diagnostischen und/oder therapeutischen Strahlungsgenerators, insbesondere einer ophthalmologischen Laservorrichtung, ausgelegt sein. Die Steuereinheit kann ferner dazu ausgelegt sein, den Strahlungsgenerator im Falle einer detektierten fehlerhaften fluidischen Kopplung der Unterdruckkavität zu deaktivieren oder abzuschalten.

In einer Ausführungsform ist die Steuereinheit dazu ausgelegt, eine fehlerhafte fluidische Kopplung der Unterdruckkavität durch Detektion einer Abweichung zwischen dem mittels dem Drucksensor ermittelten Druck und einem Referenzdruck und/oder durch Detektion eines Abfalls des Unterdrucks in Funktion der Zeit zu detektieren.

Der Referenzdruck entspricht dabei dem fest vorgegebenen und/oder einstellbaren, zeitlich konstanten und/oder zeitlich variablem Sollunterdruck zur sicheren Fixierung des Patienteninterfaces. Das Detektieren einer fehlerhaften und/oder ungenügenden Fixierung kann insbesondere durch kontinuierlichen oder quasi-kontinuierlichen Vergleich des gemessenen Unterdrucks mit dem Sollunterdruck und/oder durch Auswertung eines oder mehrerer Kennwerte einer durch eine Interpolation von Messwerten des Drucksensors gebildeten Funktion.

In einer Ausführungsform umfasst die Unterdruckvorrichtung einen mit dem der vorrichtungsseitigen Unterdruckschnittstelle fluidisch gekoppelten und mit der Steuereinheit operativ gekoppelten zweiten Drucksensor. Die Steuereinheit ist dann dazu ausgelegt, eine fehlerhafte fluidische Kopplung der Unterdruckkavität mittels Vergleichs des ermittelten Drucks mit einem vom zweiten Drucksensor ermittelten zweiten Druck zu detektieren.

Ein zweiter Drucksensor kann insbesondere mit der Unterdruckschnittstelle und dem Unterdruckgenerator fluidisch gekoppelt sein und z. B. in oder an einem Ende einer fluidischen Verbindungsleitung zwischen Unterdruckkoppler und Unterdruckgenerator oder optionalen zwischengeschalteten Ventilen angeordnet sein. Bei korrekter und durchgängiger fluidischer Verbindung zwischen Unterdruckkavität und Unterdruckvorrichtung entsprechen die vom Drucksensor und zweiten Drucksensor gemessen Drücke einander zumindest im stationären Betrieb. Eine über die Messunsicherheit hinausgehende Abweichung der gemessenen Drücke ist Indiz einer fehlerhaften Kopplung, insbesondere aufgrund eines Pseudovakuums oder einer Leckage. Die Steuereinheit kann ferner dazu ausgelegt sein, durch den Vergleich der ermittelten Drücke ein technischen Fehler eines der Drucksensoren, ihrer fluidischen und elektrischen Kopplung und/oder nachgeschalteter elektronischer Komponenten zu ermitteln, wie im Zusammenhang beispielhafter Ausführungsformen näher dargestellt. Da der Drucksensor und der zweite Drucksensor zumindest im stationären Betrieb redundant zu einander sind, kann mittels des optionalen zweiten Drucksensor ferner eine vollständige oder teilweise Blockierung oder eine Leckage der fluidischen Kopplung des Drucksensors detektiert werden.

Im stationären Betrieb der Unterdruckvorrichtung kann z. B. der Drucksensor der Überwachung dienen, während eine Regelung des Unterdruckgenerators zum Aufbau bzw. zur geregelten Aufrechterhaltung eines Unterdrucks in der Unterdruckkavität in einem den zweiten Drucksensor einschliessenden Regelkreis erfolgt. Es ist jedoch ebenfalls möglich, den Drucksensor zur Regelung zu nutzen.

In einer Ausführungsform umfasst die Unterdruckvorrichtung einen mit der vorrichtungsseitigen Unterdruckschnittstelle fluidisch gekoppelten und der Steuereinheit operativ gekoppeltem Durchflusssensor. Bei dem Durchflusssensor kann es sich beispielsweise um einen Volumenstromsensor oder einen Massenstromsensor handeln.

Die Steuereinrichtung ist in diesen Ausführungsformen zusätzlich zur Auswertung des vom Durchflusssensor ermittelten Durchflusses ausgelegt. Weitere Aspekte und Ausführungsformen unter Einschluss eines Durchflusssensors werden im Zusammenhang beispielhafter Ausführungsformen diskutiert.

In einer Ausführungsform umfasst die Unterdruckvorrichtung eine mit der vorrichtungsseitigen Unterdruckschnittstelle und dem Unterdruckgenerator fluidisch gekoppelte und mit der Steuereinheit operativ gekoppelte Ventileinheit. In einer Ausführungsform mit einer Ventileinheit ist die Ventileinheit dazu ausgelegt, die vorrichtungsseitige Unterdruckschnittstelle alternativ fluidisch abzuschliessen, fluidisch mit dem Unterdruckgenerator zu koppeln oder fluidisch mit einem Ausgleichsvolumen zu koppeln.

Eine Ventileinheit kann insbesondere der Umschaltung zwischen verschiedenen Betriebsarten bzw. Betriebszustände der Unterdruckvorrichtung dienen, so zur Umschaltung zwischen einem Absaugbetrieb zur Herstellung eines Unterdrucks in der Unterdruckkavität, einem Haltebetrieb oder stationären Betrieb zur Aufrechterhaltung des gewünschten Unterdruckes, und einem Entlüftungsbetrieb, in welchem die Unterdruckkavität bezüglich der Umgebung entlüftet und so die Fixierung des Patienteninterfaces auf dem Patientenauge aufgehoben wird. Über den funktionell separat gekoppelten Drucksensor ist ferner das Entlüften der Unterdruckkavität sicher detektierbar. Auf diese Weise ist das Vorliegen von Fehlerzuständen im Entlüftungsbetrieb, wie fehlerhafte Ventilkupplungen oder abgeknickte Schläuche etc, detektierbar. Die Steuereinheit kann optional zur Detektion derartiger weiterer Fehlerzustände ausgelegt sein.

Die Ventileinheit kann ein oder mehrere einzelne oder integral realisierte Ventil(e) umfassen und für eine separate oder gemeinsame Ansteuerung von Ventilen durch die Steuereinheit vorgesehen sein.

In einer Ausführungsform umfasst die Unterdruckvorrichtung einen Patienteninterfacekoppler, welcher sowohl die vorrichtungsseitige Unterdruckschnittstelle als auch die vorrichtungsseitige Drucksensorschnittstelle umfasst. Der Patienteninterfacekoppler ist typischerweise als lösbarer und zerstörungsfrei wieder verbindbarer fluidischer Steckverbinder oder fluidische Kupplungsbuchse mit separaten fluidischen Kanälen für die vorrichtungsseitige Unterdruckschnittstelle und die vorrichtungsseitige Drucksensorschnittstelle ausgestattet.

Gemäss eines weiteren Aspekts wird die Aufgabe durch Bereitstellung eines Patienteninterfaces gelöst. Das Patienteninterface ist zur Kopplung an ein Patientenauge ausgelegt. Das Patienteninterface umfasst eine zur fluidischen Kopplung mit dem Patientenauge ausgelegte Unterdruckkavität. Das Patienteninterface umfasst ferner eine mit der Unterdruckkavität fluidisch gekoppelte interfaceseitige Unterdruckschnittstelle zur fluidischen Kopplung der Unterdruckkavität mit einem Unterdruckgenerator. Das Patienteninterface umfasst ferner eine mit der Unterdruckkavität fluidisch gekoppelte interfaceseitige Sensorschnittstelle zur fluidischen Kopplung der Unterdruckkavität mit einem fluidischen Drucksensor. Die interfaceseitige Sensorschnittstelle ist dabei funktionell, insbesondere fluidisch, separat von der interfaceseitigen Unterdruckschnittstelle. Die fluidischen Kopplung der Unterdruckkavität mit dem Drucksensor bedeutet, dass der Drucksensor im Betrieb mit dem Druck im Innenvolumen der Unterdruckqualität beaufschlagt ist und diesen misst.

Hinsichtlich der funktionell separaten Kopplung zwischen dem Drucksensor einerseits sowie der Unterdruckkavität anderseits gelten die obigen Ausführungen. Die funktionelle und damit insbesondere fluidisch separate Kopplung des Drucksensors erstreckt sich auf den gesamten fluidischen Pfad zwischen dem Drucksensor und der interfaceseitigen Sensorschnittstelle. Die interfaceseitige Sensorschnittstelle befindet sich oder mündet unmittelbar in die und Unterdruckkavität. Der im Betrieb mit dem Patienteninterface gekoppelte Drucksensor ist dabei ein fluidischer Drucksensor wie oben beschrieben.

Das Patienteninterface kann zur Kopplung an das Patientenauge mittels Koppelflüssigkeit und/oder zur z. B. applanierenden Kopplung mittels transparenten Kontaktkörpers ausgelegt sein.

Abgesehen von der separat von der interfaceseitigen Unterdruckschnittstelle vorgesehenen interfaceseitigen Sensorschnittstelle kann das Patienteninterface in grundsätzlich bekannter Bauweise sowie ein- oder mehrteilig ausgeführt sein. Die Unterdruckkavität wird z. B. als Saugring durch eine zum Patientenauge hin offene und ringförmig umlaufende Kammer gebildet.

Beispielhafte mehrteilige Patienteninterfaces sind gemäss US20150088103A1 sowie der EP2853247A1 aufgebaut, auf welche hinsichtlich des Aufbaus beispielhafter Patienteninterfaces in das vorliegende Dokument auch für nachfolgend näher beschriebener beispielhafter Ausführungsformen einbezogen werden. Die spezifische Konstruktion des Patienteninterfaces ist jedoch nicht zwingend.

Typischerweise ist das Patienteninterface aus wirtschaftlichen Gründen sowie Sicherheitsüberlegungen heraus als Einwegprodukt ausgeführt und initial bevorzugt steril verpackt. Grundsätzlich kann das Patienteninterface aber auch ganz der in Teilen für eine mehrfache Verwendung ausgelegt sein.

In einer Ausführungsform umfasst das Patienteninterface eine Unterdruckverbindungsleitung und eine von der Unterdruckverbindungsleitung funktionell separate Drucksensorverbindungsleitung.

Die Unterdruckverbindungsleitung und die Drucksensorverbindungsleitung sind typischerweise als im erforderlichen Umfang druckbeständige und flexible und fluidisch nicht verbundene Kunststoffschläuche ausgeführt. Typischerweise sind sie an ihrem dem Patienteninterfacekörper zugewandten Ende in bekannter Weise fest mit der Unterdrucckavität fluidisch gekoppelt, z. B. über separate konische Anschlusstücke des Patienteninterfacekörpers. Ferner können die Verbindungsleitungen stoffschlüssig mit dem Patienteninterfacekörper verbunden sein, z. B. durch Kleben oder Schweissen. Alternativ können aber auch lösbare fluidische Steckverbinder am Patienteninterfacekörper zur lösbaren Verbindung mit einer separaten Unterdruckverbindungsleitung bzw. Drucksensorverbindungsleitung vorgesehen sein.

In einer Ausführungsform umfasst das Patienteninterface einen Unterdruckvorrichtungskoppler, welcher sowohl die interfaceseitige Unterdruckschnittstelle als auch die interfaceseitige Drucksensorschnittstelle umfasst. Der Unterdruckvorrichtungskoppler kann in diesen Ausführungsformen zur Kopplung mit einem Patienteninterfacekoppler einer Unterdruckvorrichtung als Gegenstück ausgelegt sein. Während die Handhabung im Betrieb durch die mechanische Integration vereinfacht wird, bleibt die fluidisch separate Kopplung hiervon unberührt.

Alternativ zu einem sowohl die interfaceseitige Unterdruckschnittstelle als auch die interfaceseitige Drucksensorschnittstelle umfassenden Koppler können aber auch separate fluidische Koppler, beispielsweise separate fluidische Steckverbinder, vorgesehen sein.

Ferner kann die Drucksensorschnittstelle auch zur unmittelbaren, bevorzugt zumindest für den Drucksensor zerstörungsfrei lösbaren Kopplung des Drucksensors ohne fluidische Drucksensorverbindungsleitung ausgelegt sein. In einer derartigen Ausführungsform ist der Drucksensor direkt am Patienteninterface bzw. Patienteninterfacekörper angekoppelt und die funktionelle Kopplung mit weiteren Elementen der Unterdruckvorrichtung, insbesondere der Steuereinheit, erfolgt über eine Kommunikationsschnittstelle, z. B. eine elektrische Leitung, welche hier an die Stelle der Drucksensorverbindungsleitung tritt. Die Drucksensorschnittstelle ist dabei unmittelbar in die Unterdruckkavität, zum Beispiel eine äußere Wandung der Unterdruckkavität, integriert bzw. mündet in diese.

In einer Ausführungsform umfasst das Patienteninterface einen fluidisch zwischen der Unterdruckkavität und der interfaceseitigen Unterdruckschnittstelle angeordneten Flüssigkeitssammler, insbesondere eine Tropfkammer. Eine derart angeordnete Tropfkammer ist aus dem Stand der Technik grundsätzlich bekannt und hält bei einem offenbarungsgemässen Patienteninterface gemeinsam mit der Luft aus der Unterdruckkavität abgesaugte Flüssigkeit zurück. Damit wird verhindert, dass diese in Kontakt mit dem nachgeordneten fluidischen System, insbesondere dem Unterdruckgenerator, dem Drucksensor und etwaigen Ventilen kommt. Diese Komponenten sind in typischen Ausführungsformen nicht für einen Flüssigkeitskontakt ausgelegt und können durch diesen beschädigt sowie z. B. Messwerte des Drucksensors verfälscht werden.

Gemäss eines weiteren Aspekts wird die Aufgabe durch Bereitstellung eines weiteren Patienteninterfaces zur Fixierung auf einem Patientenauge gelöst. Dieses Patienteninterface umfasst ein zur fluidischen Kopplung mit dem Patientenauge ausgelegtes Unterdruckkavität. Das Patienteninterface umfasst ferner eine mit der Unterdruckkavität fluidisch gekoppelte interfaceseitige Unterdruckschnittstelle zur fluidischen Kopplung der Unterdruckkavität mit einem Unterdruckgenerator. Das Patienteninterface umfasst ferner einen fluidischen Drucksensor. Der Drucksensor ist mit der Unterdruckkavität funktionell separat von der interfaceseitigen Unterdruckschnittstelle gekoppelt oder ist zur Bestimmung eines Kontaktruckes zwischen Patienteninterface und Patientenauge ausgelegt. Bei dem Drucksensor kann es sich um einen (miniaturisierten) elektronischen Drucksensor bzw. Druckwandler grundsätzlich bekannter Art, beispielsweise auf piezoresistiver Basis, handeln. Hinsichtlich der funktionell separaten Kopplung des Drucksensors an die Unterdruckkavität gelten die obigen Ausführungen.

Für diese Art von Patienteninterface ist ein Drucksensor unmittelbar in das Patienteninterface bzw. den Patienteninterfacekörper integriert. Eine hierbei zur Kopplung des Drucksensors and die Unterdruckvorrichtung vorhandene elektrische oder optische Verbindungsleitung ist typsicherweise weniger anfällig hinsichtlich Unterbrechungen, Abknicken, etc.

Zusätzlich oder alternativ zu einer operativen Verbindung zu einer Unterdruckeinheit und insbesondere ihrer Steuereinheit wie oben beschrieben kann der Drucksensor auch zur operativen Kopplung mit einer separaten Auswertevorrichtung ausgelegt sein, wobei die Auswertevorrichtung dazu ausgelegt ist, einen durch den Drucksensor ermittelten Druck auszuwerten. Diesbezügliche Funktionen der Auswertevorrichtung können im Zusammenhang der Steuereinheit einer Unterdruckvorrichtung erläuterten Funktionen entsprechen. Eine Auswertevorrichtung kann z. B. für einen autonomen Betrieb auch unmittelbar strukturell an den Patienteninterfacekörper gekoppelt und/oder in diesen integriert sein. Die Auswertevorrichtung kann Anzeigelemente und/oder Alarmierungseinrichtungen, z. B. in form analoger und/oder digitaler Anzeigen, Leuchtdioden, akustischer Signalgeber etc. umfassen, welche dazu ausgelegt sind, auf einen Fehlerzustand, beispielsweise eine fehlerhaft Kopplung des Patienteninterfaces, hinzuweisen.

Offenbarungsgemäss kann alternativ oder ergänzend zu einer Messung des Drucks bzw. Unterdrucks im Innenvolumen der Unterdruckkavität der Drucksensor auch als Kontaktdrucksensor zur Ermittlung des Kontaktrucks bzw. der Kontaktkraft zwischen Patienteninterfacekörper und Patientenauge ausgelegt sein. In derartigen Ausführungsformen ist eine zur Beaufschlagung mit dem zu messenden Druck bzw. der zu messenden Kraft vorgesehene Messfläche des Drucksensors zum Kontakt mit dem Patientenauge ausgelegt. Diese Ausführungsform ist insbesondere günstig zur Detektion einer fehlerhaften oder ungenügenden Kopplung des Patienteninterfaces an das Patientenauge, beispielsweise aufgrund einer Undichtigkeit. Der Drucksensor ist in diesem Fall ein Kontaktdrucksensor.

In einer Ausführungsform eines Patienteninterfaces mit Drucksensor umfasst der Drucksensor ein optisches Indikatorelement. Dieses variiert seine optischen Eigenschaften in Abhängigkeit eines das optische Indikatorelement beaufschlagenden Drucks.

Das optische Indikatorelement einer derartigen Ausführungsform kann z. B. ein spannungsoptisches Element sein, welches bei mechanischer Belastung in Verbindung mit einer polarisierten Lichtquelle ein die mechanische Belastung, insbesondere mechanische Spannungen, widerspiegelndes farbliches Muster zeigt. Ein derartiges optisches Indikatorelement kann beispielsweise in Form eines mit dem Innenraum der Unterdruckkavität gekoppelter Biegebalken in eine Wandlung der Unterdruckkavität, beispielsweise in eine Saugringwand, integriert sein. In einer derartigen Ausführungsform gestattet ein optisch für den Operateur zugänglicher Drucksensor bzw. optischer Indikator eine manuelle Beurteilung des Drucks in der Unterdruckkavität auch ohne zusätzliche elektronische Einrichtungen. Optional kann der Drucksensor auch eine elektronischen Sensor, beispielsweise eine miniaturisierter Kameraeinheit, Photodiode etc. umfassen ,welcher mit dem optischen Indikatorelement optisch gekoppelt ist und die die druckabhängige Änderung der optischen Eigenschaften erfasst. Ferner kann die optische Indikatoreinheit mit einer optischen Drucksensorverbindungsleitung über einen optischen Leiter, z. B. eine oder mehrere optische Fasern, verbunden werden.

Gemäss eines weiteren Aspekts wird die Aufgabe durch Bereitstellung eines Verfahrens zur Kopplung eines Patienteninterfaces an ein Patientenauge gelöst. Das Verfahren umfasst das Herstellen eines Unterdrucks in einer Unterdruckkavität des Patienteninterfaces mittels eines fluidisch an die Unterdruckkavität gekoppelten Unterdruckgenerators. Das Verfahren umfasst ferner ein Ermitteln eines Drucks in der Unterdruckkavität mittels eines vom Unterdruckgenerator funktionell separat an das Patienteninterface gekoppelten Drucksensors. Das Verfahren umfasst ferner ein Auswerten des durch den Drucksensor ermittelten Drucks. Das Auswerten des Drucks kann insbesondere das Detektieren einer fehlerhaften fluidischen Kopplung des Patienteninterfaces, wie oben sowie nachfolgend im Zusammenhang von Ausführungsbeispielen beschrieben, umfassen.

In einer Ausführungsform umfasst die Detektion einer fehlerhaften fluidischen Kopplung der Unterdruckkavität eine Detektion einer Abweichung zwischen dem mittels des Drucksensors ermittelten Drucks und eines Referenzdrucks und/oder eine Detektion eines Abfalls des Unterdrucks in Funktion der Zeit.

In einer Ausführungsform umfasst die Detektion einer fehlerhaften fluidische Kopplung der Unterdruckkavität ein Vergleichen des mittels des Drucksensors ermittelten Drucks mit einem mittels eines zweiten Drucksensors ermittelten zweiten Drucks. Dabei ist der zweite Drucksensor vom Drucksensor funktionell separat mit der Unterdruckkavität gekoppelt.

Erfindungsgemässe Verfahren können insbesondere durch bzw. unter Verwendung offenbarter Unterdruckvorrichtungen und Patienteninterfaces ausgeführt werden. Damit sind offenbarte beispielhafte Ausführungsformen von Unterdruckvorrichtungen und/oder Patienteninterfaces zugleich als Offenbarung entsprechender Ausführungsformen des Verfahrens zu verstehen, und umgekehrt.

Gemäss einem weiteren Aspekt wird die Aufgabe durch ein Computerprogrammprodukt gelöst. Das Computerprogrammprodukt umfasst ein nicht-transientes computerlesbares Medium mit darauf gespeichertem Computerprogrammcode. Der Programmcode ist dazu eingerichtet, einen oder mehrere Prozessoren einer Unterdruckvorrichtung derart zu steuern, dass
- eine Steuereinheit der Unterdruckvorrichtung einen Unterdruckgenerator der Unterdruckvorrichtung zur Herstellung eines Unterdrucks in einem mit dem Unterdruckgenerator fluidisch gekoppelten Unterdruckkavität eines Patienteninterfaces ansteuert;
- die Steuereinheit einen Druck in der Unterdruckkavität mittels eines vom Unterdruckgenerator funktionell separat an die Unterdruckkavität gekoppelten fluidischen Drucksensors ermittelt;
- die Steuereinheit den durch den Drucksensor ermittelten Druck auswertet, insbesondere eine fehlerhaft fluidische Kopplung des Patienteninterfaces detektiert.

Der eine oder die mehrere Prozessoren können insbesondere ein oder mehrere Mikroprozessoren und/oder Mikrokontroller der Steuereinheit sein. Der Computerprogrammcode kann insbesondere dazu eingerichtet sein, ein oder mehrere Prozessoren zur Ausführung einer oder mehrerer offenbarten Verfahren zu steuern.

Gemäss eines weiteren Aspekts wird die Aufgabe durch Bereitstellung einer ophthalmologischen Anordnung gelöst. Die ophthalmologische Anordnung umfasst eine Unterdruckvorrichtung und ein Patienteninterface gemäss einer der zuvor und/oder nachfolgend im Zusammenhang von Bespielen offenbarten Ausführungsform. Der Schutzbereich wird durch die Patentansprüche bestimmt.

### FIGUREN BESCHRIEB

- Figur 1: zeigt schematisch eine Ausführungsform einer ophthalmologischen Anordnung Unterdruckvorrichtung und eines Patienteninterfaces;
- Figur 2: zeigt schematisch verschieden Druckverläufe in Funktion der Zeit;
- Figur 3: zeigt schematisch eine weitere Ausführungsform einer Unterdruckvorrichtung;
- Figur 4: zeigt schematisch eine Ausführungsform eines Patienteninterfaces.
- Figur 5: zeigt schematisch eine weitere Ausführungsform einer ophthalmologischen Anordnung.

### AUSFÜHRUNGSBEISPIELE

Nachfolgend werden beispielhafte Ausführungsformen unter zusätzlichem Bezug auf die Figuren dargestellt.

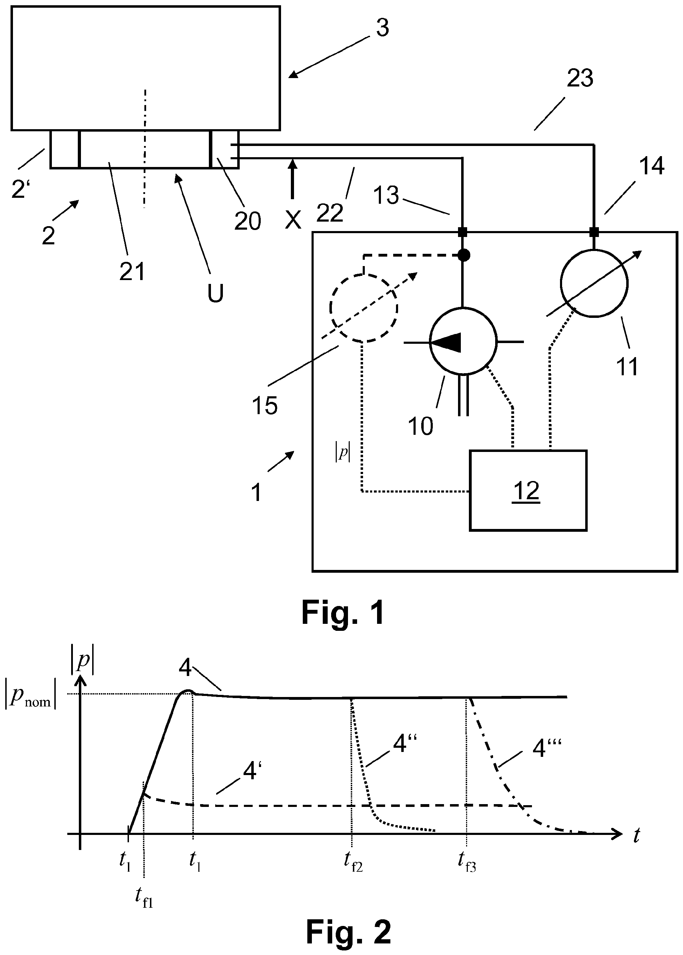

In Figur 1 bezeichnet das Bezugszeichen 1 eine Unterdruckvorrichtung und das Bezugszeichen 2 ein an die Unterdruckvorrichtung 1 angeschlossenes Patienteninterface in schematischem Querschnitt. Das Patienteninterface 2 ist mit einem ophthalmologischen Applikationskopf 3 gekoppelt. Die Unterdruckvorrichtung 1 und das Patienteninterface 2 bilden gemeinsam eine erfindungsgemässe ophthalmologische Anordnung.

Im Anwendungszustand liegt das Patienteninterface 2 mit seiner Unterseite U des Patienteninterfacekörpers 2' auf der Hornhaut des (nicht dargestellten) Patientenauges auf. Der Patienteninterfacekörper 2' des Patienteninterfaces 2 weist einen zum Beispiel zylindrischen Innenraum 21 auf, welcher sich im Anwendungszustand zwischen der Hornhautoberfläche des Patientenauges und dem Applikationskopf 3 befindet und beispielsweise mit physiologische Kochsalzlösung als Koppelflüssigkeit gefüllt sein kann. Konzentrisch um den Innenraum 21 ist ein Saugring angeordnet, welcher im Anwendungszustand ebenfalls auf der Hornhaut des Patientenauges aufliegt und dessen Innenraum eine ringförmige Unterdruckkavität 20 bildet. Zur Kopplung des Patienteninterfaces 2 bzw. des Patienteninterfacekörpers 2' an das Patientenauge wird in der Unterdruckkavität 20 ein Unterdruck bzw. Vakuum erzeugt, welches so das Patienteninterface 2 auf dem Patientenauge fixiert.

Die Unterdruckvorrichtung 1 umfasst einen Unterdruckgenerator 10, der typischerweise durch eine Vakuumpumpe gebildet wird. Die Unterdruckvorrichtung 1 umfasst ferner einen fluidischen Drucksensor 11 und eine mit dem Unterdruckgenerator 10 und dem Drucksensor 11 operativ verbundene Steuereinheit 12, welche typischerweise durch eine elektrische/elektronische Schaltung grundsätzlich bekannter Art gebildet wird. Insbesondere kann die Steuereinheit 12 einen oder mehrere Mikroprozessoren und/oder Mikrokontroller mit entsprechendem Programmcode zur Steuerung der Funktion der Unterdruckvorrichtung 2. Die Steuereinheit 12 umfasst eine Alarmierungsvorrichtung oder ist mit einer (nicht dargestellten) Alarmierungsvorrichtung operativ gekoppelt. Durch die z. B. optische und/oder akustische Alarmierungsvorrichtung erfolgt eine Alarmierung im Falle einer festgestellten fluidischen Kopplung. Die Steuereinheit 12 kann ferner einen mit dem Applikationskopf 3 verbundenen oder im Applikationskopf 3 enthaltene ophthalmologische Laserlichtquelle oder sonstigen Strahlengenerator operativ gekoppelt sein und diesen im Falle einer detektierten fehlerhaften fluidischen Kopplung der Unterdruckkavität deaktivieren oder abschalten.

Die Unterdruckvorrichtung 1 umfasst ferner eine vorrichtungsseitige Unterdruckschnittstelle 13 sowie eine vorrichtungsseitige Drucksensorschnittstelle 14, welche zum Beispiel durch lösbare fluidische Steckverbinder oder Koppler, z. B. fluidische Kupplungsbuchsen, gebildet werden. Dabei ist die vorrichtungsseitige Unterdruckschnittstelle 13 fluidisch mit dem Unterdruckgenerator 10 und die vorrichtungsseitige Drucksensorschnittstelle 14 separat mit dem Drucksensor 11 fluidisch gekoppelt.

Das Patienteninterface 2 umfasst ferner eine Unterdruckverbindungsleitung 22 sowie eine von der Unterdruckverbindungsleitung 22 fluidisch separate Drucksensorverbindungsleitung 23. Die Unterdruckverbindungsleitung 22 und die Drucksensorverbindungsleitung 23 sind mit einem Ende jeweils separat fluidisch an die Unterdruckkavität 20 gekoppelt, wobei sich die fluidisch separate Kopplung jeweils auf den gesamten fluidischen Pfad und insbesondere bis zur Unterdruckkavität 20 erstreckt. Am jeweils anderen Ende weisen die Unterdruckverbindungsleitung 22 und die Drucksensorverbindungsleitung 23 jeweils einen fluidisches Koppelelement (nicht separat dargestellt), z. B. einen fluidische Steckverbinder auf, der zur lösbaren Kopplung mit der vorrichtungsseitigen Unterdruckschnittstelle 13 bzw. der vorrichtungsseitigen Drucksensor Schnittstelle 14 vorgesehen ist.

Zur Kopplung des Patienteninterfaces 2 an das Patientenauge wird der Unterdruckgenerator 10 durch die Steuereinheit 12 angesteuert bzw. in Betrieb gesetzt, so das das in der Unterdruckkavität 20 ursprünglich vorhandene Luft zumindest teilweise abgesaugt wird. Der funktionell separat mit der Unterdruckkavität 20 gekoppelte Drucksensor 11 misst den effektiv in der Unterdruckkavität 20 vorhandenen Druck unabhängig von der Unterdruckverbindungsleitung 22.

Nachfolgend wird zusätzlich auf Figur 2 Bezug genommen. In Figur 2 stellt die mit dem Bezugszeichen 4 gekennzeichnete Kurve den Betrag des Unterdrucks *p* , so wie er vom Drucksensor 11 gemessen wird, als Funktion der Zeit *t* für eine korrekte Fixierung des Patienteninterfaces 2 auf dem Patientenauge dar. Dabei entspricht ein Wert von *p* = 0 dem Umgebungsdruck und ein zunehmender Unterdruck (abnehmender Absolutdruck) einem ansteigenden Kurvenverlauf.

In der zum Zeitpunkt *t*₀ beginnenden Absaugphase wird in der Unterdruckkavität 20 vorhandene Luft durch den Unterdruckgenerator 10 durch die Unterdruckverbindungsleitung 22 abgesaugt, wodurch der Unterdruck ansteigt. Bei einem dem vorgesehenen Betriebszustand entsprechenden Sollunterdruck von z. B. *p*ₙₒₘ = -400mbar schaltet die Steuereinheit 12 zum Zeitpunkt *t*₁ in bekannter Weise zu einen stationären Haltebetrieb um, bei welchem der Unterdruck in der Unterdruckkavität 20 im Wesentlichen konstant gehalten wird. Dieser Zustand bleibt erhalten, bis bewusst ein Druckausgleich mit der Umgebung hergestellt und so das Patienteninterface 2 von der Hornhaut des Patientenauges gelöst wird.

Der beim Absaugen der Luft aus dem Unterdruckvolumen 20 entstehende Luftstrom aus dem Unterdruckvolumen heraus in Richtung der Unterdruckvorrichtung 1 bzw. des Unterdruckgenerators 10 bewirkt zudem eine Entfernung etwaiger vorhandener Flüssigkeitströpfchen, Resten von Sterilabdeckfolie etc. aus dem Bereich der Kopplung zwischen Drucksensorverbindungsleitung 23 und Unterdruckvolumen 20.

Die Kurve 4' in Figur 2 zeigt schematisch den durch den Drucksensor 11 gemessenen Druck *p* , wenn zu einem ersten Fehlerzeitpunkt Zeitpunkt *t*_{f1} ein Pseudovakuum, wie in der allgemeinen Beschreibung dargestellt, entsteht, zum Beispiel durch Abknicken der Unterdruckverbindungsleitung 22 während des Ansaugvorgangs an einer in Figur 1 beispielhaft mit "X" markierten Stelle. Ein in der Unterdruckvorrichtung 1 an die Unterdruckverbindungsleitung 22 angeschlossener Drucksensor 15, wie er nach dem Stand der Technik typischerweise vorhanden und bei der in Figur 1 gezeigten Vorrichtung optional ist, kann das Abknicken der Unterdruckverbindungsleitung 22 nicht detektieren, da zwischen dem Ort des Abknickens und der Unterdruckvorrichtung 1 bzw. dem Unterdruckgenerator 10 weiter ein Unterdruck vorhanden ist bzw. auch weiter aufgebaut wird, und so vom Drucksensor 15 ein Unterdruck gemessen wird, der jedoch in der Unterdrucckavität 20 nicht vorhanden ist.

Aufgrund der unmittelbaren und von der Unterdruckverbindungsleitung 22 unabhängigen Kopplung des Drucksensors 11 an die Unterdruckkavität 20 misst der Drucksensor dagegen 11 den in der Unterdruckkavität 20 effektiv vorhanden Druck. Entsprechend steigt der vom Drucksensor 11 gemessene Unterdruck nach dem Abknicken der Unterdruckverbindungsleitung 20 nicht weiter an, obwohl der Unterdruckgenerator 10 weiterarbeitet. Der Unterdruck in der Unterdruckkavität 20 bleibt nach dem Auftreten des Pseudovakuums (auf zu geringem Niveau) im Wesentlichen konstant oder fällt aufgrund von Elastizität und/oder gegebenenfalls vorhandenen Leckagen wieder ab, so dass ein Druckausgleich mit der Umgebung stattfindet.

Die Kurve 4" in Figur 2 stellt den vom Drucksensor 11 gemessenen Druck für den Fall dar, dass der Unterdruck in der Unterdruckkavität 20 (nach einem zunächst korrekt erfolgten Aufbau des Unterdrucks und einer korrekten Fixierung des Patienteninterfaces 2 auf dem Patientenauge) zu einem zweiten Fehlerzeitpunkt *t*_{f2} während des stationären Betriebs abfällt. Dies ist zum Beispiel der Fall, wenn sich das Patienteninterface 2 vom Patientenauge kurzfristig und teilweise löst, was in ungünstigen Fällen, z. B. aufgrund einer erforderlichen Bewegung des Patienteninterfaces durch den Augenarzt oder auch eine Bewegung des Patienten selbst, erfolgen kann. In diesem Fall findet ein zumindest teilweiser Druckausgleich mit der Umgebung und damit einen Abfall des Unterdrucks in der Unterdruckkavität 20 statt. Grundsätzlich kann dieser Druckabfall durch den Drucksensor 15 detektiert werden und der Unterdruckgenerator 10 den korrekten Unterdruck mindestens im Fall einer nur kurzzeitigen Leckage wiederherstellen. Ist jedoch die Unterdruckverbindungsleitung 22 selbst abgeknickt oder aus anderem Grunde nicht durchgängig, wird der Abfall des Unterdrucks durch den Drucksensor 15 nicht erkannt. Bei einer nur teilweise durchgängigen Unterdruckverbindungsleitung 22 erhöht sich zumindest die Regelverzögerung beim Ausregeln des Druckabfalls, so dass eine sichere Fixierung des Patienteninterfaces 2 auf dem Patientenauge unter Umständen ebenfalls nicht mehr gegeben ist oder zumindest nicht gewährleistet werden kann. Der erfindungsgemäss unmittelbar an die Unterdruckkavität 20 angeschlossener Drucksensor 11 stellt den Abfall des Unterdrucks gemäss Kurve 4" korrekt fest.

Die Kurve 4‴ in Figur 2 stellt den Fall dar, dass (nach einem zunächst korrekt erfolgten Aufbau des Unterdrucks und einer korrekten Fixierung des Patienteninterfaces 2 auf dem Patientenauge) der Unterdruck zu einem dritten Fehlerzeitpunkt *t*_{f3} (im Vergleich zum in Kurve 4" dargestellten Verlauf) langsam abfällt, was z. B. durch eine geringfügige Leckage oder Undichtigkeit eines fluidischen Verbinders bedingt werden kann.

Die Feststellung von Fehlerzuständen hinsichtlich der Fixierung des Patienteninterfaces 2 auf dem Patientenauge durch Auswertung des vom Drucksensor 11 gemessenen Drucks bzw. des dem Druck entsprechenden elektrischen Signals kann durch die Steuervorrichtung 12 alternativ oder sich ergänzend nach verschiedenen Verfahren erfolgen, welche typischerweise durch einen oder mehrere Mikrokontroller/oder Prozessoren der Steuervorrichtung 12 mit entsprechendem Programmcode/Firmware ausgeführt werden. Ein oder mehrere Verfahren können jedoch ganz oder teilweise auch durch spezielle Hardware und entsprechende Schaltungen realisiert sein.

Nach einem Verfahren wird der durch den Drucksensor 11 gemessene Druck kontinuierlich oder quasi-kontinuierlich mit mindestens einem Grenzunterdruck *p*ₗᵢₘᵢₜ verglichen. Dieser Grenzdruck *p*ₗᵢₘᵢₜ liegt typischerweise betragsmässig tiefer als der Sollunterdruck *p*ₙₒₘ und kann z. B. durch den Betrag des Sollunterdruck *p*ₙₒₘ abzüglich eines durch Toleranzen, Messunsicherheit etc. bestimmten Sicherheitswertes erfolgen. In der Absaugphase kann der Grenzdruck *p*ₗᵢₘᵢₜ entsprechend dem sich bei korrektem Betrieb ergebenden Druckverlauf kontinuierlich angepasst werden. Ein Fehlerfall wird dann bei Unterschreitung des Grenzdrucks *p*ₗᵢₘᵢₜ angenommen.

Nach einem weiteren alternativen oder ergänzenden Verfahren wird der durch den Drucksensor 11 gemessene Druck kontinuierlich oder quasi-kontinuierlich hinsichtlich eines Abfalls bzw. Nachlassen des Unterdrucks ausgewertet. Hierfür können zahlreiche grundsätzlich bekannte Verfahren der Signalverarbeitung und/oder Statistik zum Einsatz kommen, beispielsweise die Bestimmung und Auswertung der Steigung und/oder weiterer Kennwerte einer durch eine Interpolation von Messwerten gebildeten Funktion.

Umfasst die Unterdruckvorrichtung 1 zusätzlich den optionalen zweiten Drucksensor 15 kann nach einem weiteren alternativen oder ergänzenden Verfahren die Detektion einer fehlerhaften oder unzureichenden Fixierung durch einen Vergleich bzw. eine gemeinsame Auswertung der durch den Drucksensor 11 und den zweiten Drucksensor 15 ermittelten Drücke erfolgen. Im Falle einer korrekten Kopplung des Patienteninterfaces 2 und ohne das Vorliegen eines Fehlerzustandes sind die vom Drucksensor 11 und dem zweiten Drucksensor 15 gemessenen Drücke mindestens im stationären Betriebszustand im Wesentlichen gleich und die Sensoren 11, 15 damit redundant. Entsprechend kann die Detektion einer fehlerhaften oder ungenügenden fluidischen Kopplung des Patienteninterfaces die Detektion einer Abweichung zwischen den durch den Drucksensor 11 bzw. den zweiten Drucksensor 15 ermittelten Drücken umfassen. Das Ermitteln der Abweichung kann dabei mittels grundsätzlich bekannter Verfahren der Signalverarbeitung und/oder Statistik erfolgen und z. B. einer Ermittlung und Auswertung einer Differenz der ermittelten Drücke und den Vergleich mit einer zulässigen Höchstdifferenz umfassen, welche sich typischerweise aus Toleranzen und Messunsicherheiten bestimmt. Alternativ oder ergänzend zu einer Differenzbildung kann das Feststellen einer fehlerhaften oder ungenügenden Kopplung beispielsweise das Ermitteln und Auswerten einer Korrelation der durch den Drucksensor 11 und den zweiten Drucksensor 15 ermittelten Drücke in Funktion der Zeit *t* umfassen.

Bei einer gemeinsamen Auswertung der durch den Drucksensor 11 und den zweiten Drucksensor 15 ermittelten Drücke ist zu beachten, dass der Drucksensor 11 insbesondere während des Aufbaus des Unterdrucks in der Unterdruckkavität 20 und beim Entlüften auf Druckänderungen in der Unterdruckkavität 20 schneller reagiert als der zweite Unterdrucksensor 15. Dies ergibt sich daraus, dass der über die Unterdruckverbindungsleitung 22 zugleich eine Verschiebung von Luftvolumen erfolgt, während die Drucksensorverbindungsleitung 23 durch den Drucksensor 11 fluidisch abgeschlossen ist.

Die Steuervorrichtung 12 kann ferner dazu ausgelegt sein, verschiedene Verfahren zur Detektion einer fehlerhaften und/oder ungenügenden Fixierung parallel oder alternativ anzuwenden.

Durch eine gemeinsame Auswertung der durch den Drucksensor 11 und den zweiten Drucksensor 15 ermittelten Drücke kann die Steuervorrichtung 12 ferner weitere Fehlerzustände detektieren, so ein Pseudovakuum der Drucksensorverbindungsleitung 23 und/oder einen Defekt eines von Drucksensor 11 und zweitem Drucksensor 15, seiner elektrischen Kontaktierung oder nachgeschalteter Komponenten.

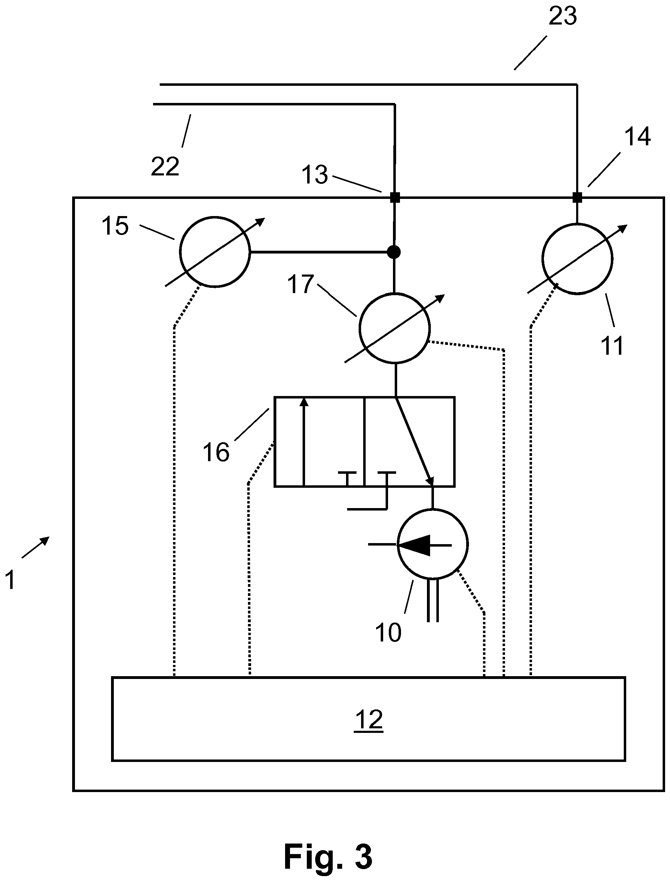

Nachfolgend wird zusätzlich auf Figur 3 Bezug genommen. Figur 3 zeigt ein weiteres Ausführungsbeispiel der Unterdruckvorrichtung 1. Die Unterdruckvorrichtung 1 aus Figur 3 ist in grundsätzlich ähnlicher Weise aufgebaut wie die Unterdruckvorrichtung 1 gemäss Figur 1, enthält jedoch zusätzlich eine operativ mit der Steuereinheit gekoppelte und durch die Steuereinheit 12 angesteuerte Ventileinheit 16.

Über die Ventileinheit 16 wird die Unterdruckversorgungsleitung 16 alternativ mit dem Unterdruckgenerator 10 (dargestellte Stellung) zu Aufbau und Aufrechterhaltung des Unterdrucks oder mit der Umgebung zur Entlüftung und zum Abbau des Unterdrucks in der Unterdruckkavität 20 verbunden.

Die in Figur 3 dargestellte Ausführungsform der Unterdruckvorrichtung 11 umfasst ferner einen optionalen Durchflusssensor 17, welcher fluidisch zwischen der vorrichtungsseitigen Unterdruckschnittstelle 13 und der Ventileinheit angeordnet ist. Der Durchflusssensor 17 dient der Detektion von Fehlerzuständen, insbesondere von temporären oder dauerhaften Leckagen, welche beim Absaugen von Luft aus der Unterdruckkavität 20 einen erhöhten Luftfluss bewirken. Ferner bewirkt das Vorhandensein eines Pseudovakuums gemäss der zuvor beschriebenen Art, dass bei Betrieb des Unterdruckgenerators 10 der zweite Drucksensor 15 zwar einen Unterdruck misst, jedoch im Vergleich zu zur Situation ohne Pseudovakuum kein oder nur ein reduzierter Luftdurchfluss durch den Durchflusssensor 17 erfolgt.

Die in Figur 1 und Figur 3 dargestellten Ausführungsformen einer Unterdruckvorrichtung 1 können in verschiedener Weise modifiziert werden. So kann der Drucksensor 11 anstelle innerhalb eines Gehäuses (nicht referenziert) der Steuereinheit 1 unmittelbar am bzw. im Patienteninterface 2 angeordnet sein, wobei die Drucksensorverbindungsleitung 23 entfällt und stattdessen eine elektrische Drucksensor-Anschlussleitung vorgesehen ist. In derartigen Ausführungsformen kann der Drucksensor 11 beispielsweise ein miniaturisierter Einweg-Drucksensor sein, welcher z. B. fest mit dem Patienteninterfacekörper 2' verbaut ist, oder der Patienteninterfacekörper 2' bzw. die Unterdruckkavität 20 sowieso der Drucksensor 11 weisen eine zumindest für den Drucksensor 11 zerstörungsfrei lösbare fluidische Schnittstelle auf.

Die vorrichtungsseitige Unterdruckschnittstelle 13 sowie die vorrichtungsseitige Drucksensorschnittstelle 14 können sich anstatt an einem Gehäuse der Unterdruckvorrichtung 1 auch an der Schnittstelle zum Patienteninterfacekörper 2' befinden. In diesem Fall können die Unterdruckverbindungsleitung 22 und die Drucksensorverbindungsleitung 23 ganz oder teilweise Teil der Unterdruckeinrichtung 1 sein. Die fluidischen Schnittstellen 13, 14 können mittels separater fluidischer Koppler, beispielsweise separater fluidischer Steckverbinder, realisiert werden, oder in einen gemeinsamen fluidischen Koppler oder Steckverbinder integriert sein.

Der Aufbau, insbesondere der fluidisch Aufbau, der Unterdruckvorrichtung 1, kann ferner modifiziert werden und insbesondere weitere Komponenten umfassen. So kann die Ventilanordnung 16 gemäß Figur 3 weitere Ventile umfassen und weitere fluidische Konfiguration ermöglichen. So kann beispielsweise vorgesehen werden, den Unterdruckgenerator 10 mit der Umgebung fluidisch zu verbinden. Ferner kann vorgesehen werden, die Unterdruckanschlussleitung 22 z. B. gemeinsam mit dem angeschlossenen zweiten Drucksensor 15 fluidisch abzuschliessen bzw. fluidisch zu isolieren. Ferner kann ein Unterdruckreservoir mit einem Volumen in einem Bereich von z. B. einem Liter vorgesehen kann, welches mittels der Ventilanordnung mit der Unterdruck-Versorgungsleitung 22 und/oder dem Unterdruckgenerator 10 verbindbar ist. Ein solches Unterdruckreservoir dient insbesondere der fluidischen Pufferung und kann ferner an Stelle und in grundsätzlich gleicher Funktion wie der Unterdruckgenerator zum Absaugen kleiner Luftmengen, beispielsweise im Falle einer kleineren und kurzzeitigen Leckage der Unterdruckkavität 20. Der Aufbau eines Unterdrucks im optionalen Unterdruckreservoir erfolgt vorteilhaft mittels des Unterdruckgenerators 10.

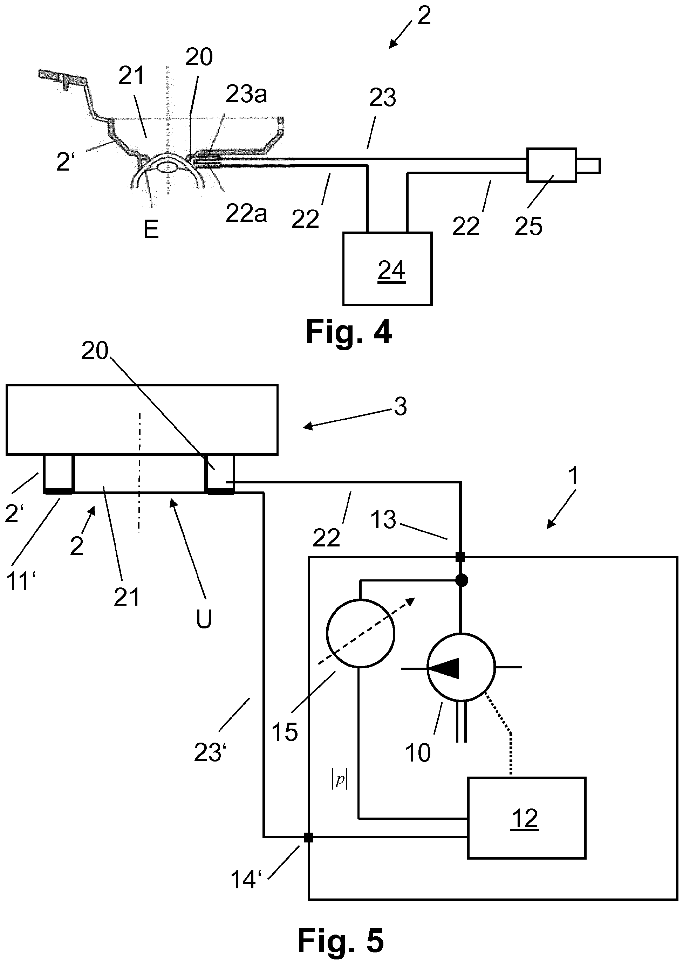

Nachfolgend wird zusätzlich auf Figur 4 Bezug genommen. Figur 4 stellt schematisch einen Teil eines Patienteninterfaces 2 gemäss einer beispielhaften Ausführungsform der Erfindung gemeinsam mit einem Patientenauge E dar. Das Patienteninterface 2 besitzt einen Patienteninterfacekörper 2' mit einer ringförmigen Unterdruckkavität 20 und einem Innenraum 21. In die Unterdruckkavität münden separat zwei fluidische Anschlussstutzen 22a, 23a, welche mit den patienteninterfaceseitigen Ende der Unterdruckanschlussleitung 22 und der Drucksensor-Anschlussleitung 23 in grundsätzlich bekannter Weise fluidisch dicht verbunden sind, z. B. durch Kleben, Ultraschall-Schweissen, oder durch Reibschluss. Die vorrichtungsseitigen Enden der fluidischen Leitungen 22, 23 münden in einen gemeinsamen Unterdruckvorrichtungskoppler 25 in Form eines Steckverbinders, der im Betrieb mit einem entsprechenden Patienteninterfacekoppler der Unterdruckvorrichtung 1 in Form einer fluidischen Kupplungsbuchse gekoppelt wird. Der Unterdruckvorrichtungskoppler 25 und der zugehörige Patienteninterfacekoppler sind Doppelverbinder, welche für die gemeinsame Herstellung bzw. Lösung zweier fluidisch separater Verbindungen ausgelegt wird. Wenngleich ein derartiger Doppelverbinder unter Gesichtspunkten der Betriebssicherheit sowie der Handhabung vorteilhaft ist, können alternativ auch zwei separate Koppler, z. B. einfache fluidische Steckverbinder, vorgesehen sein.

Das Patienteninterface 2 gemäss Figur 4 umfasst ferner eine optional in die Unterdruckverbindungsleitung 22 eingeschleifte Tropfkammer 24. Die Tropfkammer scheidet von der Unterdruckkavität 20 abgesaugte Flüssigkeit, beispielsweise Tropfen von physiologischer Kochsalzlösung als Koppelflüssigkeit, ab und verhindert so, dass diese im Betrieb in die fluidischen Komponenten der Unterdruckvorrichtung 1 gelangen.

Da die Tropfkammer 24 fluidisch zwischen der Unterdruckkavität 20 und dem zweiten Drucksensor 15 angeordnet ist, verlangsamt sie das Ansprechen des zweiten Drucksensors 1 5 bzw. erhöh seine Trägheit. Dies gilt jedoch nicht für den direkt mit dem Unterdruckvolumen 20 gekoppelten Drucksensor 11, der somit auf Druckänderungen im Unterdruckvolumen 20 schneller reagiert.

Nachfolgend wird zusätzlich auf Figur 5 Bezug genommen. Figur 5 stellt eine weitere Ausführungsform einer ophthalmologischen Anordnung mit einer weiteren Ausführungsform der Unterdruckvorrichtung 1 und einer weitere Ausführungsform des Patienteninterfaces 2 in einer schematischen funktionellen Darstellung und in operativ gekoppeltem Zustand dar. Soweit nicht nachfolgend erwähnt, können die Unterdruckvorrichtung 1 und das Patienteninterface 2 nach Figur 5 analog zur Darstellung gemäss Figur 1 aufgebaut sein und entsprechende Funktionalität besitzen.

In der Ausführungsform gemäß Figur 5 ist der fluidische Drucksensor 11 durch einen Kontaktdrucksensor 11' ersetzt, welcher im Patienteninterface 2 angeordnet ist. Der Kontaktdrucksensor 1 1' ist zur Messung des Kontraktdrucks zwischen Patienteninterface 2 und dem Patientenauge ausgelegt und beispielhaft ringförmig in die dem Patientenauge zugewandten Seite einer Wandung des Saugrings integriert. Alternativ können auch mehrere isolierte Kontaktdrucksensoren entlang des Umfangs des Saugrings oder auch nur ein einzelner isolierter Kontaktdrucksensor vorgesehen sein.

Die fluidische Drucksensorverbindungsleitung 23 der Figur 1 ist in dieser Form durch eine elektrische Drucksensor Verbindungsleitung 23' ersetzt, über welche der Kontaktdrucksensor 11' mit der Unterdruckvorrichtung 1 funktionell elektrisch gekoppelt wird. Entsprechend ist statt der fluidischen vorrichtungsseitigen Drucksensorschnittstelle 14 gemäß Figur 1 eine elektrische vorrichtungsseitige Drucksensorschnittstelle 14', z. B. in Form eines elektrischen Steckverbinders, vorgesehen. Ein fluidische Drucksensor 1 1 gemäß Figur 1 kann optional zusätzlich vorgesehen sein.

## Patentansprüche

1. Unterdruckvorrichtung (1) zur Fixierung eines Patienteninterfaces (2) auf einem Patientenauge, die Unterdruckvorrichtung (1) umfassend:
- einen Unterdruckgenerator (10) und eine vorrichtungsseitigen Unterdruckschnittstelle (13) zur fluidischen Kopplung des Unterdruckgenerators (10) an eine Unterdruckkavität (20) des Patienteninterfaces (2);
- einen fluidischen Drucksensor (11) und eine vorrichtungsseitige Drucksensorschnittstelle (14) zur von der vorrichtungsseitigen Unterdruckschnittstelle (13) funktionell separaten Kopplung des Drucksensors (11) an das Patienteninterface (2);
- eine mit dem Unterdruckgenerator (10) und dem Drucksensor (11) operativ gekoppelte Steuereinheit (12), wobei die Steuereinheit (12) dazu ausgelegt ist, den Unterdruckgenerator (10) zur Erzeugung eines Unterdrucks in der Unterdruckkavität (20) anzusteuern sowie ferner dazu ausgelegt ist, einen durch den Drucksensor (11) ermittelten Druck auszuwerten.

2. Unterdruckvorrichtung (1) nach Anspruch 1, wobei die Steuereinheit (12) dazu ausgelegt ist, eine fehlerhafte fluidische Kopplung der Unterdruckkavität (20) durch Detektion einer Abweichung zwischen dem mittels des Drucksensors (11) ermittelten Druck und einem Referenzdruck und/oder durch Detektion eines Abfalls des Unterdrucks in Funktion der Zeit zu detektieren.

3. Unterdruckvorrichtung (1) nach einem der Ansprüche 1 oder 2, wobei die Unterdruckvorrichtung (1) einen mit der vorrichtungsseitigen Unterdruckschnittstelle (13) fluidisch gekoppelten und mit der Steuereinheit (12) operativ gekoppelten zweiten Drucksensor (15) umfasst, und wobei die Steuereinheit (12) dazu ausgelegt ist, eine fehlerhafte fluidische Kopplung der Unterdruckkavität (20) mittels Vergleichs des ermittelten Drucks mit einem vom zweiten Drucksensor (15) ermittelten zweiten Druck zu detektieren.

4. Unterdruckvorrichtung (1) nach einem der Ansprüche 1 bis 3, wobei die Unterdruckvorrichtung (1) einen mit der vorrichtungsseitigen Unterdruckschnittstelle (13) fluidisch gekoppelten und der Steuereinheit (12) operativ gekoppelten Durchflusssensor (17) zur Ermittlung eines Fluidstroms durch die vorrichtungsseitige Unterdruckschnittstelle (13) umfasst.

5. Unterdruckvorrichtung (1) nach einem der Ansprüche 1 bis 4, wobei die Unterdruckvorrichtung (1) eine mit der vorrichtungsseitigen Unterdruckschnittstelle (13) und dem Unterdruckgenerator (10) fluidisch gekoppelte und mit der Steuereinheit (12) operativ gekoppelte Ventileinheit (16) umfasst.

6. Unterdruckvorrichtung (1) nach Anspruch 5, wobei die Ventileinheit (16) dazu ausgelegt ist, die vorrichtungsseitige Unterdruckschnittstelle (13) alternativ fluidisch abzuschliessen, fluidisch mit dem Unterdruckgenerator (10) zu koppeln oder fluidisch mit einem Ausgleichsvolumen zu koppeln.

7. Unterdruckvorrichtung (1) nach einem der Ansprüche 1 bis 6, wobei die Unterdruckvorrichtung (1) einen Patienteninterfacekoppler umfasst, wobei der Patienteninterfacekoppler sowohl die vorrichtungsseitige Unterdruckschnittstelle (13) als auch die vorrichtungsseitige Drucksensorschnittstelle (14) umfasst.

8. Patienteninterface (2) zur Fixierung auf einem ein Patientenauge, das Patienteninterface (2) umfassend:
- eine zur fluidischen Kopplung mit dem Patientenauge ausgelegtes Unterdruckkavität (20);
- eine mit der Unterdruckkavität (20) fluidisch gekoppelte interfaceseitige Unterdruckschnittstelle zur fluidischen Kopplung der Unterdruckkavität (20) mit einem Unterdruckgenerator (10);
- eine mit der Unterdruckkavität (20) fluidisch gekoppelte interfaceseitige Sensorschnittstelle zur fluidischen Kopplung der Unterdruckkavität (20) mit einem fluidischen Drucksensor (11), wobei die interfaceseitige Sensorschnittstelle funktionell separat von der interfaceseitigen Unterdruckschnittstelle ist.

9. Patienteninterface (2) nach Anspruch 8, wobei das Patienteninterface (2) eine Unterdruckverbindungsleitung (22) und eine von der Unterdruckverbindungsleitung (22) funktionell separate Drucksensorverbindungsleitung (23) umfasst.

10. Patienteninterface (2) nach einem der Ansprüche 8 bis 9, wobei das Patienteninterface (2) einen Unterdruckvorrichtungskoppler (25) umfasst, wobei der Unterdruckvorrichtungskoppler (25) sowohl die interfaceseitige Unterdruckschnittstelle als auch die interfaceseitige Drucksensorschnittstelle umfasst.

11. Patienteninterface (2) nach einem der Ansprüche 8 bis 10, wobei das Patienteninterface (2) einen fluidisch zwischen der Unterdruckkavität (20) und der interfaceseitigen Unterdruckschnittstelle (13) angeordneten Flüssigkeitssammler, insbesondere eine Tropfkammer (24), umfasst.

12. Verfahren zur Kopplung eines Patienteninterfaces (2) an ein Patientenauge, das Verfahren umfassend:
- Herstellen eines Unterdrucks in einer Unterdruckkavität (20) des Patienteninterfaces (2) mittels eines an fluidisch an die Unterdruckkavität (20) gekoppelten Unterdruckgenerators (10);
- Ermitteln eines Drucks in der Unterdruckkavität (20) mittels eines vom Unterdruckgenerator (10) funktionell separat an das Patienteninterface (2) gekoppelten fluidischen Drucksensors (11);
- Auswerten des mittels des durch den Drucksensors (11) ermittelten Drucks.

13. Ophthalmologische Anordnung, umfassend eine Unterdruckvorrichtung (1) gemäss einem der Ansprüche 1 bis 7 und ein Patienteninterface (2) gemäss einem der Ansprüche 8 bis 11.

## Claims

1. Negative pressure device (1) for affixing a patient interface (2) to a patient's eye, the negative pressure device (1) including:
- a negative pressure generator (10) and a device-side negative pressure interface (13) for fluidically coupling the negative pressure generator (10) to a negative pressure cavity (20) of the patient interface (2);
- a fluidic pressure sensor (11) and a device-side pressure sensor interface (14) for coupling the pressure sensor (11) to the patient interface (2) in a manner functionally separate from the device-side negative pressure interface (13);
- a control unit (12) operatively coupled to the negative pressure generator (10) and the pressure sensor (11), the control unit (12) being configured to control the negative pressure generator (10) to generate negative pressure in the negative pressure cavity (20) and further being configured to evaluate a pressure determined by the pressure sensor (11).

2. Negative pressure device (1) according to claim 1, wherein the control unit (12) is configured to detect defective fluidic coupling of the negative pressure cavity (20) by detecting a deviation between the pressure determined via the pressure sensor (11) and a reference pressure and/or by detecting a reduction in the negative pressure as a function of time.

3. Negative pressure device (1) according to claims 1 or 2, wherein the negative pressure device (1) includes a second pressure sensor (15) that is fluidically coupled to the device-side negative pressure interface (13) and operatively coupled to the control unit (12) and wherein the control unit (12) is configured to detect defective fluidic coupling of the negative pressure cavity (20) by comparing the determined pressure with a second pressure determined by the second pressure sensor (15).

4. Negative pressure device (1) according to any one of claims 1 to 3, wherein the negative pressure device (1) comprises a flow sensor (17) that is fluidically coupled to the device-side negative pressure interface (13) and operatively coupled to the control unit (12) and that serves to determine a fluid flow through the device-side negative pressure interface (13).

5. Negative pressure device (1) according to any one of claims 1 to 4, wherein the negative pressure device (1) includes a valve unit (16) that is fluidically coupled to the device-side negative pressure interface (13) and the negative pressure generator (10) and operatively coupled to the control unit (12).

6. Negative pressure device (1) according to claim 5, wherein the valve unit (16) is configured to alternatively fluidically seal the device-side negative pressure interface (13), fluidically couple said device-side negative pressure interface to the negative pressure generator (10) or fluidically couple said device-side negative pressure interface to a compensation volume.

7. Negative pressure device (1) according to any one of claims 1 to 6, wherein the negative pressure device (1) includes a patient interface coupler, wherein the patient interface coupler includes both the device-side negative pressure interface (13) and the device-side pressure sensor interface (14).

8. Patient interface (2) for affixation to a patient's eye, the patient interface (2) including:
- a negative pressure cavity (20) configured for fluidic coupling to the patient's eye;
- an interface-side negative pressure interface which is fluidically coupled to the negative pressure cavity (20) and which serves to fluidically couple the negative pressure cavity (20) to a negative pressure generator (10) ;
- an interface-side sensor interface which is fluidically coupled to the negative pressure cavity (20) and serves to fluidically couple the negative pressure cavity (20) to a fluidic pressure sensor (11), the interface-side sensor interface being functionally separate from the interface-side negative pressure interface.

9. Patient interface (2) according to claim 8, wherein the patient interface (2) includes a negative pressure connecting line (22) and a pressure sensor connecting line (23) that is functionally separate from the negative pressure connecting line (22).

10. Patient interface (2) according to either of claims 8 and 9, wherein the patient interface (2) includes a negative pressure device coupler (25), the negative pressure device coupler (25) including both the interface-side negative pressure interface and the interface-side pressure sensor interface.

11. Patient interface (2) according to any one of claims 8 to 10, wherein the patient interface (2) includes a liquid collection means, in particular a droplet chamber (24), fluidically arranged between the negative pressure cavity (20) and the interface-side negative pressure interface (13).

12. Method for coupling a patient interface (2) to a patient's eye, the method including:
- establishing negative pressure in a negative pressure cavity (20) of the patient interface (2) with a negative pressure generator (10) that is fluidically coupled to the negative pressure cavity (20);
- determining a pressure in the negative pressure cavity (20) by a fluidic pressure sensor (11) that is coupled to the patient interface (2) in a manner functionally separate from the negative pressure generator (10);
- evaluating the pressure determined by the pressure sensor (11).

13. Ophthalmological arrangement including a negative pressure device (1) according to any one of claims 1 to 7 and a patient interface (2) according to any one of claims 8 to 11.

## Revendications

1. Dispositif à pression négative (1) destiné à fixer une interface patient (2) sur un œil d'un patient, le dispositif à pression négative (1) comprenant:
- un générateur de pression négative (10) et une interface à pression négative côté dispositif (13) destinée à coupler fluidiquement le générateur de pression négative (10) à une cavité à pression négative (20) de l'interface patient (2);
- un capteur de pression fluidique (11) et une interface de capteur de pression côté dispositif (14) destinée à effectuer un couplage, séparé fonctionnellement de l'interface à pression négative côté dispositif (13), du capteur de pression (11) à l'interface patient (2);
- une unité de commande (12) couplée opérationellement au générateur de pression négative (10) et au capteur de pression (11), l'unité de commande (12) étant conçue pour commander le générateur de pression négative (10) afin de générer une pression négative dans la cavité à pression négative (20) et étant conçue en outre pour évaluer une pression déterminée par le capteur de pression (11).

2. Dispositif à pression négative (1) selon la revendication 1, l'unité de commande (12) étant conçue pour détecter un couplage fluidique défectueux de la cavité à pression négative (20) en détectant un écart entre la pression déterminée par le capteur de pression (11) et une pression de référence et/ou en détectant une chute de pression négative en fonction du temps.

3. Dispositif à pression négative (1) selon l'une des revendications 1 ou 2, le dispositif à pression négative (1) comprenant un deuxième capteur de pression (15) couplé fluidiquement à l'interface à pression négative côté dispositif (13) et couplé opérationellement à l'unité de commande (12), et l'unité de commande (12) étant conçue pour détecter un couplage fluidique défectueux de la cavité à pression négative (20) en comparant la pression déterminée à une deuxième pression déterminée par le deuxième capteur de pression (15).

4. Dispositif à pression négative (1) selon l'une des revendications 1 à 3, le dispositif à pression négative (1) comprenant un capteur de débit (17) couplé fluidiquement à l'interface à pression négative côté dispositif (13) et couplé opérationellement à l'unité de commande (12) afin de déterminer un flux de fluide à travers l'interface à pression négative côté dispositif (13).

5. Dispositif à pression négative (1) selon l'une des revendications 1 à 4, le dispositif à pression négative (1) comprenant une unité formant soupape (16) couplée fluidiquement à l'interface à pression négative côté dispositif (13) et au générateur de pression négative (10) et couplée opérationellement à l'unité de commande (12).

6. Dispositif à pression négative (1) selon la revendication 5, l'unité formant soupape (16) étant conçue pour alternativement isoler fluidiquement l'interface à pression négative côté dispositif (13), la coupler fluidiquement au générateur de pression négative (10) ou la coupler fluidiquement à un volume de compensation.

7. Dispositif à pression négative (1) selon l'une des revendications 1 à 6, le dispositif à pression négative (1) comprenant un coupleur d'interface patient, le coupleur d'interface patient comprenant l'interface à pression négative côté dispositif (13) ainsi que l'interface de capteur de pression côté dispositif (14).

8. Interface patient (2) destinée à la fixation sur l'œil d'un patient, l'interface patient (2) comprenant:
- une cavité à pression négative (20) conçue pour effectuer un couplage fluidique à l'œil du patient;
- une interface à pression négative côté interface qui est couplée fluidiquement à la cavité à pression négative (20) et qui est destinée à coupler fluidiquement la cavité à pression négative (20) à un générateur de pression négative (10);
- une interface de capteur côté interface qui est couplée fluidiquement à la cavité à pression négative (20) et qui est destinée à coupler fluidiquement la cavité à pression négative (20) à un capteur de pression fluidique (11), l'interface de capteur côté interface étant fonctionnellement séparée de l'interface à pression négative côté interface.

9. Interface patient (2) selon la revendication 8, l'interface patient (2) comprenant une ligne de liaison à pression négative (22) et une ligne de liaison de capteur de pression (23) fonctionnellement séparée de la ligne de liaison à pression négative (22).

10. Interface patient (2) selon l'une des revendications 8 à 9, l'interface patient (2) comprenant un coupleur de dispositif à pression négative (25), le coupleur de dispositif à pression négative (25) comprenant l'interface à pression négative côté interface ainsi que l'interface de capteur de pression côté interface.

11. Interface patient (2) selon l'une des revendications 8 à 10, l'interface patient (2) comprenant un collecteur de liquide, en particulier une chambre compte-gouttes (24), disposé fluidiquement entre la cavité à pression négative (20) et l'interface à pression négative côté interface (13).

12. Procédé de couplage d'une interface patient (2) à un œil d'un patient, le procédé comprenant:
- générer une pression négative dans une cavité à pression négative (20) de l'interface patient (2) au moyen d'un générateur de pression négative (10) couplé fluidiquement à la cavité à pression négative (20);
- déterminer une pression dans la cavité à pression négative (20) au moyen d'un capteur de pression fluidique (11) couplé à l'interface patient (2) de manière fonctionnellement séparée du générateur de pression négative (10);
- évaluer la pression déterminée par le capteur de pression (11).

13. Ensemble ophtalmologique, comprenant un dispositif à pression négative (1) selon l'une des revendications 1 à 7 et une interface patient (2) selon l'une des revendications 8 à 11.
